(19) **Europäisches Patentamt**
**European Patent Office**
**Office européen des brevets**

(11) **EP 4 380 460 B1**

(12) # EUROPEAN PATENT SPECIFICATION

(45) Date of publication and mention
of the grant of the patent:
**13.11.2024 Bulletin 2024/46**

(21) Application number: **22754423.6**

(22) Date of filing: **26.07.2022**

(51) International Patent Classification (IPC):
**A61B 8/08** (2006.01)    **A61B 8/00** (2006.01)
**A61B 8/06** (2006.01)

(52) Cooperative Patent Classification (CPC):
**A61B 8/0891; A61B 8/469; A61B 8/5246;**
**A61B 8/5269; A61B 8/54; G01S 7/52033;**
**G01S 7/52066; G01S 15/8915; G01S 15/8979;**
**G06T 5/92;** A61B 8/06; A61B 8/4209; A61B 8/4236;
A61B 8/4494; A61B 8/488;            (Cont.)

(86) International application number:
**PCT/EP2022/070921**

(87) International publication number:
**WO 2023/011983 (09.02.2023 Gazette 2023/06)**

(54) **METHOD AND SYSTEM FOR ULTRASOUND PARAMETER IDENTIFICATION**

VERFAHREN UND SYSTEM ZUR IDENTIFIZIERUNG VON ULTRASCHALLPARAMETERN

PROCÉDÉ ET SYSTÈME D'IDENTIFICATION DE PARAMÈTRES ULTRASONORES

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **03.08.2021 EP 21189288**

(43) Date of publication of application:
**12.06.2024 Bulletin 2024/24**

(73) Proprietor: **Koninklijke Philips N.V.**
**5656 AG Eindhoven (NL)**

(72) Inventors:
• **SHULEPOV, Sergei Y.**
**5656 AG Eindhoven (NL)**
• **VAN ROOIJ, Johannes Antonius**
**5656 AG Eindhoven (NL)**
• **PALANISAMY, Krishnamoorthy**
**5656 AG Eindhoven (NL)**
• **JOCHEMSEN, Robert**
**5656 AG Eindhoven (NL)**
• **VAN KESTEREN, Hans Willem**
**5656AG Eindhoven (NL)**

(74) Representative: **Philips Intellectual Property &**
**Standards**
**High Tech Campus 52**
**5656 AG Eindhoven (NL)**

(56) References cited:
JP-A- 2000 139 914       US-A1- 2008 154 131
US-A1- 2008 242 982       US-A1- 2010 069 755
US-A1- 2011 054 317       US-A1- 2012 123 265

• **MOSHAVEGH RAMIN ET AL: "Advanced**
**automated gain adjustments for in-vivo**
**ultrasound imaging", 2015 IEEE**
**INTERNATIONAL ULTRASONICS SYMPOSIUM**
**(IUS), IEEE, 21 October 2015 (2015-10-21), pages**
**1 - 4, XP032799287, DOI:**
**10.1109/ULTSYM.2015.0298**
• **KUMAR AMIT ET AL: "An Automatic**
**Segmentation Algorithm of Boundary Layers**
**from B-Mode Ultrasound Images", 2018 RECENT**
**ADVANCES ON ENGINEERING, TECHNOLOGY**
**AND COMPUTATIONAL SCIENCES (RAETCS),**
**IEEE, 6 February 2018 (2018-02-06), pages 1 - 6,**
**XP033391579, DOI:**
**10.1109/RAETCS.2018.8443959**

(52) Cooperative Patent Classification (CPC): (Cont.)
    G06T 2207/10132; G06T 2207/20008;
    G06T 2207/30104

**Description**

FIELD OF THE INVENTION

The invention relates to the field of ultrasound parameter identification, and more particularly to the field of identifying parameter values for ultrasound hemodynamic analysis.

BACKGROUND OF THE INVENTION

In ultrasound diagnostic imaging, typical hemodynamic measurements of a blood vessel are performed by skilled sonographers, wherein the sonographer positions an ultrasound image plane in the middle of the vessel and along its main axis. Typically, the sonographer adjusts ultrasound parameter values, in order to realize an adequate ultrasound image for hemodynamic analysis.

Recently, wearable ultrasound patches for the assessment of arterial hemodynamics have been realised. Such ultrasound patches prove to be reliable, robust, and non-invasive. However, measurements performed by the ultrasound patch are often strongly dependent on position (and reposition) of the patch. This may prove problematic if the operator uses non-optimal preset parameter values for the ultrasound patch, resulting in a poor quality data stream, and an unreliable assessment of hemodynamic parameters.

Indeed, ultrasound patches are often not linked to a real-time visualization system. As such, it may not be immediately apparent to the operator that the selected ultrasound parameters, or the placement of the ultrasound patch, is non-optimal. Indeed, manual tuning of the ultrasound parameters is often not possible without a system for visualization.

Moshavegh et al. (2015) for example disclose in "Advanced Automated Gain Adjustments for In-Vivo Ultrasound Imaging" an advanced and automated gain adjustment method that compensates for the gains on scans and dynamically adapts to the drastic attenuation variations between different media.

US 2008/0154131 discloses a method for imaging during ablation procedures using ultrasound imaging is provided.

JP 2000/139914 discloses a luminance regulator that makes a luminance adjustment, i.e., a gain adjustment, and a contrast adjustment according to a luminance adjustment curve inside a region of interest.

US 2010/0069755 discloses an ultrasound diagnostic apparatus comprising an image constructing section for producing a tomographic image frame composed of brightness information based on an intensity of a receive signal, and adjusting brightness of the produced tomographic image frame with a set gain.

US 2012/0123265 discloses embodiments for enhancing an image quality of an ultrasound image based on entropy information.

US 2008/0242982 discloses a method to reduce noise manifest in blood vessel ultrasound B-mode images based on blood flow information.

Further, it has been demonstrated that for a more reliable and robust automatic assessment of the arterial hemodynamics, the ultrasound patch should be applied crossing the vessel under a certain angle. However, such an angled measurement with respect to the arteries imposes higher demands on the quality of ultrasound image. Specifically, edges of the artery will refract the ultrasound waves, and may not be fully rendered. Thus, an even more specific set of parameter values for the ultrasound patch will need to be applied to ensure adequate ultrasound image quality.

SUMMARY OF THE INVENTION

The invention is defined by the independent claims. The dependent claims define advantageous embodiments.

According to examples in accordance with an aspect of the invention, there is provided a method for identifying a gain parameter value for ultrasound hemodynamic analysis, the method comprising obtaining a B-mode ultrasound image of a blood vessel; determining a global B-mode gain value which results in a global image entropy of the B-mode ultrasound image being within a target entropy range; identifying a region of interest of the B-mode ultrasound image resulting from the global B-mode gain value; and determining an output B-mode gain value is at least based on a contrast-to-noise ratio of the region of interest.

Proposed is a method for determining a B-mode gain value, which may provide B-mode ultrasound images for accurate hemodynamic analysis. The method may first determine a global B-mode gain value having an adequate entropy, and may then refine this B-mode gain value locally in a region of interest to achieve a satisfactory contrast-to-noise ratio.

Specifically, in order to ascertain a region of interest, there must be enough information present in the B-mode ultrasound image. This is achieved by first determining a global B-mode gain value which delivers a B-mode ultrasound image within a target entropy range. Once the region of interest is identified, then local improvement of the B-mode gain may occur. This is based on a contrast-to-noise ratio in the region of interest. The contrast-to noise ratio may be maximized, ensuring the best image quality in the region of interest.

The output B-mode gain value may be used to obtain B-mode ultrasound images of a blood vessel of a subject. Such B-mode ultrasound images may facilitate accurate, stable, and robust hemodynamic analysis.

In other words, it has been realized that changing the B-mode gain value such that the entire B-mode ultrasound image demonstrates good overall average quality may not deliver the best results for hemodynamic analysis. In many cases, it leads to poor imaging of the blood vessel region, as the contrast of the surrounding tissue will be optimized instead. More specifically, features of the blood vessel wall (such as intima and media thickness) may not be resolved sufficiently well. Instead, by changing the B-mode gain value such that the image is locally optimized, more stable and accurate B-mode ultrasound images of the blood vessel may be provided for hemodynamic analysis. While this may reduce image quality in the surrounding area, it has been realized that imaging the surrounding area is largely irrelevant for hemodynamic analysis.

The proposed method(s) may be performed automatically, and in real time. Advantageously, this obviates the need for a skilled operator to manually tune parameter values to obtain an ultrasound image of the blood vessel of the subject. In many circumstances, is not possible for a skilled operator to manually tune the parameters, as feedback is not available.

In some embodiments, the obtained B-mode ultrasound image may result from an initial B-mode gain value, and determining the global B-mode gain value may comprise: calculating the global image entropy of the B-mode ultrasound image; and determining the global B-mode gain value by adjusting the initial B-mode gain value, the global B-mode gain resulting in the global image entropy of the B-mode ultrasound image being within the target entropy range.

Typically, a standard preset B-mode gain value is applied to the ultrasound, which may not be appropriate for the given subject and/or blood vessel. In this case, the initial B-mode gain value may be this standard preset. By adjusting from this initial B-mode gain value, a global B-mode gain value delivering a B-mode ultrasound image having a global image entropy within the target entropy range may be found, and may be found more quickly than from a random B-mode gain value.

In some embodiments, adjusting the initial B-mode gain value to determine the global B-mode gain value may comprise one of increasing the global B-mode gain value from the initial B-mode gain value until the global image entropy of the B-mode ultrasound image resulting from the B-mode gain value is within the target entropy range; or decreasing the global B-mode gain value from the initial B-mode gain value until the global image entropy of the B-mode ultrasound image resulting from the B-mode gain value is within the target range.

In this way, the B-mode gain value may be gradually changed until the global image entropy is within the target entropy range. By gradually increasing or decreasing the B-mode gain value from an initial B-mode gain value, the lowest and/or highest B-mode gain value may be found which delivers a global image entropy within the target entropy range.

In some embodiments, the obtained B-mode ultrasound image may result from an initial B-mode gain value, and determining the global B-mode gain value may comprise: plotting a cumulative histogram of image entropy of the B-mode ultrasound image resulting from the initial B-mode gain value; determining the global B-mode gain value by adjusting the initial B-mode gain value, such that a log-average of the image entropy of the B-mode ultrasound image resulting from the global B-mode gain is within the target entropy range.

A straightforward and effective method for determining a global B-mode gain value that may provide a B-mode ultrasound image with a global image entropy within the target entropy range is to plot a cumulative histogram. This method may be performed in real-time, as it is fast, and computationally inexpensive.

In some embodiments, the target entropy range may be a range of log-averages of the cumulative histogram, the range of log-averages selected such that a common carotid artery, CCA, of corresponding B-mode ultrasound images may be detected.

By selecting the range of log-averages such that the CCA may be detected, a global B-mode gain value may be determined which is suitable for providing a B-mode ultrasound image in which a region of interest may be quickly and consistently identified.

In some embodiments, identifying the region of interest may comprise obtaining color/power Doppler data corresponding to the B-mode ultrasound image resulting from the global B-mode gain value; identifying the blood vessel in the B-mode ultrasound image resulting from the global B-mode gain value based on the obtained color/power Doppler data; and determining the region of interest based on a diameter of the blood vessel and a direction of the blood vessel.

One method of determining the region of interest is by leveraging color/power Doppler data to select the relevant blood vessel. This color/power Doppler data may be obtained alongside the B-mode ultrasound images, and therefore it may require no additional computation to obtain - saving time and processing power. Then, from the blood vessel direction and diameter, a region of interest may be determined. This region of interest may be stable and accurate, ultimately leading to an improved output B-mode gain value.

In some embodiments, determining the output B-mode gain value may comprise calculating the contrast-to-noise ratio of the region of interest of the B-mode ultrasound image resulting from the global B-mode gain value; and determining the output B-mode gain value by adjusting the global B-mode gain value to result in an increase in the contrast to noise ratio of the region of interest.

The global B-mode gain value may provide a B-mode ultrasound image which can act as a starting point for local image improvement. Specifically, the contrast-to-noise ratio in the region of interest of this B-mode ultrasound image may be determined, and the B-mode gain value adjusted accordingly to increase the contrast-to-noise ratio. Thus, an improved B-mode ultrasound image in the region of interest may be obtained, leading to more accurate hemodynamic analysis.

In some embodiments, determining the output B-mode gain value may comprise identifying a value of the output B-mode gain value which results in a maximized contrast to noise ratio in the region of interest.

In this way, an output B-mode gain may be identified which is best suited for the individual subject and blood vessel of interest. This gain value may be used to obtain B-mode ultrasound images in which hemodynamic analysis may be accurate and stable.

In some embodiments, calculating the contrast-to-noise ratio may be based on a difference between an average intensity of pixels in the region of interest corresponding to a tissue wall of the blood vessel and an average intensity of pixels in the region of interest corresponding to a background region, and calculating the contrast-to-noise ratio may be further based on a variance of intensity of pixels in the background region of the region of interest.

In particular, pixel intensity of the walls of the blood vessel compared to a background region should be maximized in order to improve the accuracy of hemodynamic analysis. However, the variance of intensity of the pixels in the background region should also be reduced. Typically, variance in intensity of the pixels associated with the walls is useful, in order to capture features of interest. Therefore, the variance in intensity of the wall pixels is not included in the noise term. In this way, a B-mode ultrasound image acquired using the output B-mode gain value may be more useful for hemodynamic analysis.

In some embodiments, the method may further comprise checking if the output B-mode gain value the global image entropy of the B-mode ultrasound image resulting from the output B-mode gain value is within the target entropy range.

By further checking that the output B-mode gain value may be used to produce a B-mode ultrasound image with sufficient entropy, improved accuracy hemodynamic analysis may be achieved.

According to another aspect of the invention, there is provided a computer program product comprising computer program instruction or code which is adapted, when said computer program is run on a computer, to implement the method for identifying a gain parameter value for ultrasound hemodynamic analysis.

According to yet another aspect of the invention, there is provided a control system for identifying a gain parameter value for ultrasound hemodynamic analysis, the control system comprising: an interface configured to obtain a B-mode ultrasound image of a blood vessel; an entropy analysis unit configured to determine a global B-mode gain value which results in a global image entropy of the B-mode ultrasound image being within a target entropy range; an image analysis unit configured to identify a region of interest of the B-mode ultrasound image resulting from the global B-mode gain value; and a contrast analysis unit configured to determine an output B-mode gain value based on a contrast-to-noise ratio of the region of interest.

According to a further aspect of the invention, there is provided a system for ultrasound hemodynamic analysis of a subject, the system comprising: a control system for identifying a gain parameter value according to an aspect of the invention; and an ultrasound monitor configured to acquire a B-mode ultrasound image of a blood vessel of the subject at the output B-mode gain value determined by the control system.

By functionally connecting the control system to an ultrasound monitor, a B-mode gain value of the ultrasound monitor may be determined by the control system and used to directly control the ultrasound monitor in real-time.

In some embodiments, the ultrasound monitor may comprise a wearable ultrasound sensor or an ultrasound patch.

Usually, wearable ultrasound monitors are less intrusive and uncomfortable compared to typical ultrasound monitors. As a result, subject comfort may be improved while undergoing hemodynamic analysis.

In some embodiments, the ultrasound monitor comprises a one-dimensional ultrasonic array.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 depicts a configuration and output of standard hemodynamic analysis of a blood vessel;
Figure 2 depicts an operation of identification of an optimal cross-section between an ultrasound imaging plane and the blood vessel;

Figure 3A shows a number of B-mode ultrasound images of a blood vessel with different user applied B-mode gain values;

Figure 3B shows the results of edge detection for the user applied B-mode gains of Figure 3A;

Figure 4 is a flow diagram of a method for identifying a gain parameter value for ultrasound hemodynamic analysis according to an exemplary embodiment;

Figure 5 is a flow diagram of a method for determining a global B-mode gain according to an aspect of an exemplary embodiment;

Figure 6 depicts various stages of histogram equalization of an ultrasound image of a blood vessel;

Figure 7 is a flow diagram of a method for determining a region of interest of an ultrasound image according to an aspect of an exemplary embodiment;

Figure 8A and 8B depict the identification of a region of interest of an ultrasound image;

Figure 9 is a flow diagram of a method for determining an output B-mode gain value according to an aspect of an exemplary embodiment;

Figure 10A and 10B depict the impact of a change in the contrast-to noise ratio on the measurement of a diameter of a blood vessel;

Figure 11 shows the results of a measurement of a diameter of a blood vessel as a B-mode gain value is varied;

Figure 12A, 12B and 12C present B-mode ultrasound images acquired using two user defined B-mode gain values, and one using an automatically obtained output B-mode gain value;

Figure 13 shows a simplified block diagram of a system for identifying a gain parameter value for ultrasound hemodynamic analysis according to an exemplary embodiment;

Figure 14 shows a simplified block diagram a system for ultrasound hemodynamic analysis of a subject according to an exemplary embodiment.

DETAILED DESCRIPTION OF THE EMBODIMENTS

The invention will be described with reference to the Figures.

It should be understood that the detailed description and specific examples, while indicating exemplary embodiments of the invention, are intended for purposes of illustration only and are not intended to limit the scope of the invention. These and other features, aspects, and advantages of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to".

Implementations in accordance with the present disclosure relate to various techniques, methods, schemes and/or solutions pertaining to the identification of a gain parameter value for ultrasound hemodynamic analysis by determining a global B-mode gain value, identifying a region of interest, and adjusting the global B-mode gain value to improve the contrast-to-noise ratio in the region of interest. According to proposed concepts, a number of possible solutions may be implemented separately or jointly. That is, although these possible solutions may be described below separately, two or more of these possible solutions may be implemented in one combination or another.

Embodiments of the invention aim to provide a method of identifying a B-mode gain value that may be used for accurate hemodynamic analysis. In particular, global improvement of the B-mode gain is performed such that a global B-mode gain is determined which is used to obtain a B-mode ultrasound image having a global image entropy within a target entropy range. Thus, this image may have enough entropy, or information, in order to identify a region of interest of the image. This region of interest may be, for example, a common carotid artery (CCA), or another blood vessel of the subject. Local image improvement is then performed based on the contrast-to-noise ratio in the region of interest.

Accordingly, the method may be used to ascertain a B-mode gain value for an ultrasound monitor for hemodynamic monitoring (HDM). This may be performed automatically, without the need for manual tuning by a trained professional, while still being suitable for hemodynamic monitoring. Indeed, it may often be the case that a trained professional cannot manually tune the ultrasound parameters, as there exists no feedback mechanism.

In other words, the embodiments of the invention may enable an automated improvement/optimization of the ultrasound pre-set parameters for accurate assessment of arterial hemodynamics of a subject by means of a non-invasive, ultrasound-based HDM patch.

Looking at the invention another way, the selection of suitable ultrasound parameter values may vary depending on both the subject, and the individual blood vessel of interest. While it may initially seem sufficient to simply to maximize the entropy of the ultrasound image obtained by adjusting ultrasound parameters, this may result in an improved contrast of the surrounding tissue, while reducing image quality of the blood vessel region. Indeed, features of the blood vessel wall such as intima and media thickness may not be clear. Therefore, by further optimizing the image locally based on a contrast-to-noise ratio of a region of interest, a more accurate, stable, and robust HDM system may be realized.

By way of explanation, continuous and accurate blood flow measurements of a blood vessel are essential in HDM applications. However, reliability of the automatic hemodynamic quantifications depends on the quality of underlying ultrasound data streams. Thus, it is of great importance to optimize ultrasound parameter settings in such a system individually for each patient. This should be realized, as much as possible, without intervention of the operator in order to avoid any operator dependent variability.

Typically, a standard vendor optimized pre-set is selected for monitoring a subject. Put another way, initial ultrasound parameter values prescribed by the manufacturer of the ultrasound machine are initially used for monitoring a subject. Using these initial parameter values, the ultrasound image of the patient is visualized. Based on this visualization, the sonographer adjusts the ultrasound parameter values to optimize acquisition for the particular patient, taking into account specificity of patient's physiology.

Indeed, it is clear that selection of the ultrasound parameter values requires significant training and experience of the sonographer, which may not be be available in monitoring environment. Moreover, a real time data visualization requires a state-of-the art hardware equipment, which may not directly fit into current workflow/monitoring practices. As such, the identification of ultrasound parameter variables should be fully automated, while still accounting for specificity of the patient physiology.

Thus, some embodiments of the proposed invention may include the following main components:

a pre-set, model-based, automatic adaptation of the image histogram to have optimal information (entropy) of the image for the artery size assessment;
an automatic selection of the ROI relevant for the assessment of hemodynamic parameters; and
a model-based optimization of the ultrasound B-mode gain to ensure stable, operator and motion independent quantitative estimation of the hemodynamic parameters.

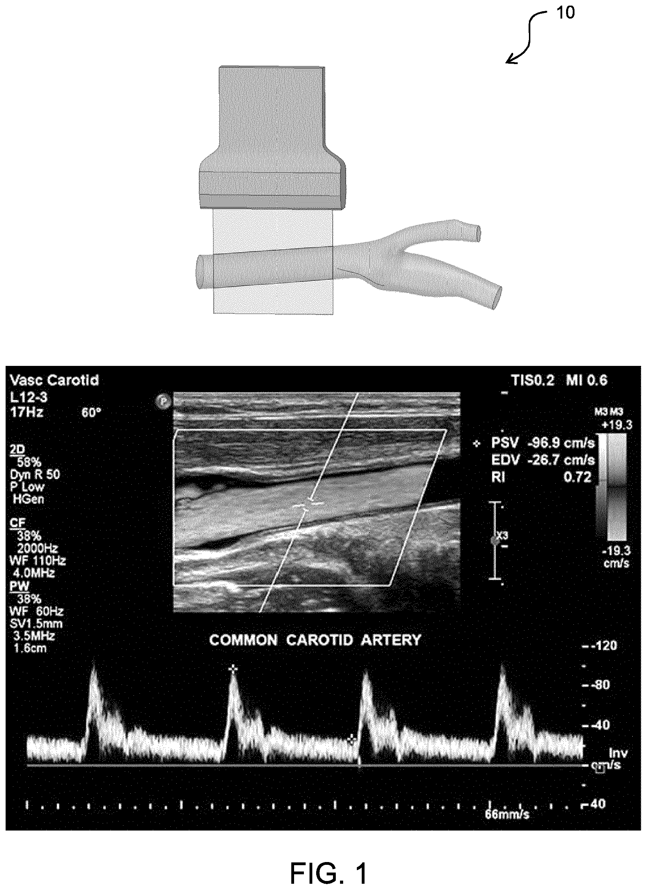

Turning now to Figure 1, a typical configuration and output for hemodynamic analysis of a blood vessel of a subject 10 is depicted. Specifically, a ultrasound plane is placed across a blood vessel by a skilled professional. The output is shown, which includes a profile of blood flow through the CCA with time.

Typically, monitoring of blood flow includes:

qualitatively determining the presence/absence of blood flow; and
hemodynamic quantification, such as blood velocity, artery diameter, volumetric flow rate, and derivatives.

In order to determine such results, hemodynamic monitoring of the blood vessels usually involves a combination of structural imaging (B-mode imaging) and color/power Doppler imaging (color flow, spectral Doppler).

To put a common workflow of hemodynamic monitoring simply, firstly the patch is applied by a user/operator with guidance provided by indicators. Indicators may be placed on the patch, and/or may be displayed on the monitor.

Initially, standard carotid preset parameter values are applied to B-mode imaging and color/power Doppler imaging. These include a multi-region TGC, a B-mode gain, color/power Doppler gain and scale settings. The preset parameter values are universal, and so are very unlikely to be optimal for a specific subject. However, these parameters are usually good enough to deliver an ultrasound image in which discrimination between main carotid blood vessels is possible.

Currently, a manual fine tuning of the B-mode image is further realized to assure the highest echo quality in the regions where properties of the vessels are to be evaluated. However, in the monitoring setup images can be either not available, or operator not skilled enough to perform such image optimization. Therefore, embodiments of the invention may provide an automatic procedure to achieve this goal, specifically for the monitoring application.

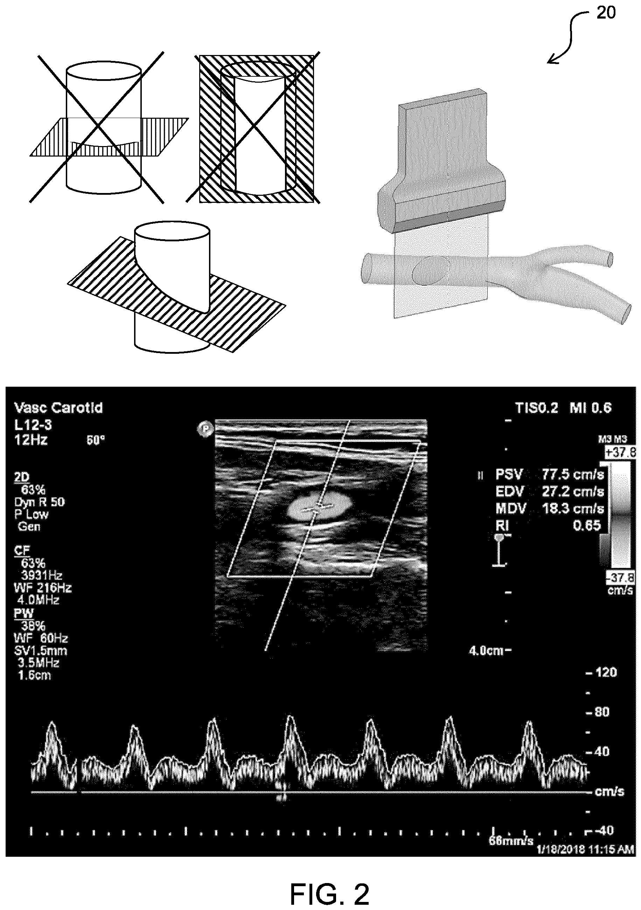

Figure 2 depicts an operation 20 of identification of an optimal cross-section between an ultrasound imaging plane and the blood vessel, and placement of the ultrasound scanner.

In particular, guidance of placing the ultrasound patch for the artery sizing is based on a combination of B-mode and color/power Doppler ultrasound data. It is ensured that the transverse elements of the ultrasound patch are well below the bifurcation (about 2 cm down from the vessel bifurcation). This is to ensure minimal effect of turbulent flow near the bifurcation in estimating blood flow parameters. Indeed, placement guiding may also be available without displaying the actual ultrasound image on the monitor screen, allowing a non-trained user to appropriately apply the monitoring device.

It has recently been demonstrated that for a more reliable, and robust automatic assessment of the arterial hemodynamics, the ultrasound-based HDM patch should be applied crossing the blood vessel under a certain, optimal angle. The angled probe allows the subject to move more freely, while still rendering all the hemodynamic parameters with accuracy of the standard assessment method illustrated in Figure 1.

However, monitoring using an ultrasound patch angled with respect to the blood vessel imposes higher demands on the quality of ultrasound image. Specifically, edges of the blood vessel refract the ultrasound waves, and as such may not be fully rendered. By adjusting the B-mode gain of the signal in the region of interest, additional features of the tissue can be either suppressed or rendered with more details. There exists a challenge in optimizing ultrasound settings for a given patient, allowing for stable and accurate hemodynamic parameter identification without intervention of the operator. Some embodiments of the invention aim to overcome this challenge.

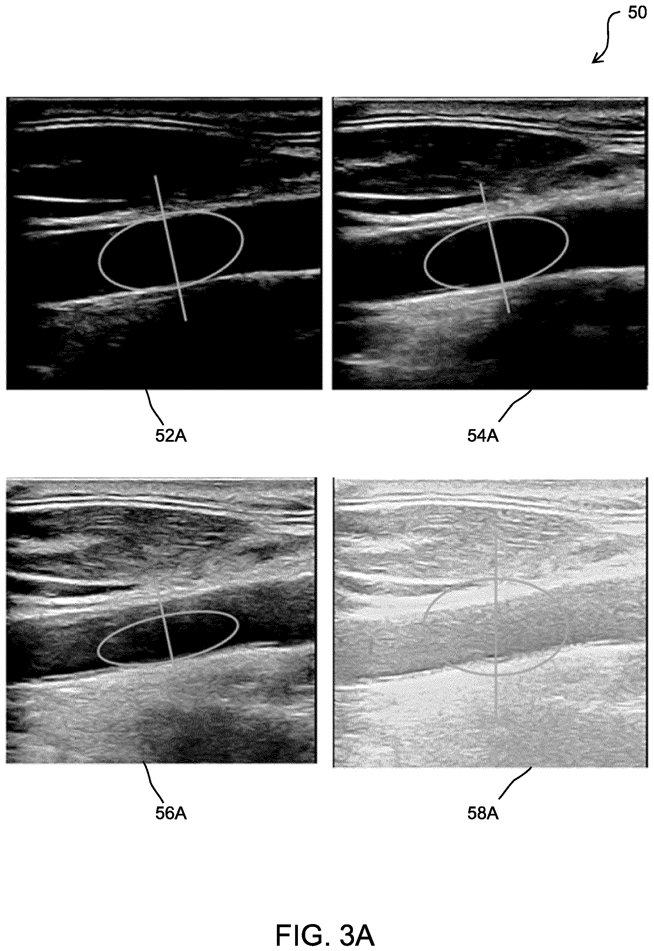

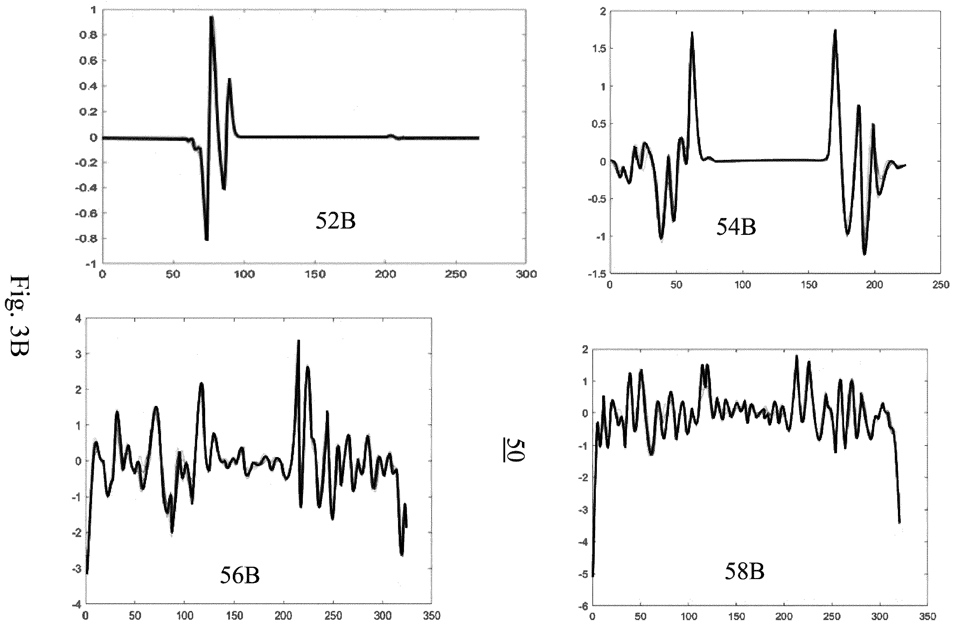

Figure 3A shows a number of B-mode ultrasound images of a blood vessel with different user applied B-mode gain values. Figure 3B shows results of edge detection of the blood vessels shown in Figure 3A. In particular, Figure 3B shows a graph of the intensity of pixel values along the center lines of the blood vessels identified in Figure 3B.

More specifically, each of 52A, 54A, 56A and 58A show the B-mode ultrasound image of a CCA at different user defined gains. Results of an edge detection algorithm for each image are presented in 52B, 54B, 56B and 58B. It is clear that the change in B-mode gain values results in a varied estimation of blood vessel diameter. In cases shown in 52A and 58A, reliable detection of the blood vessel diameter under dynamic conditions may not be possible, or may require significantly post-processing. In the case shown in 56A, the diameter may be determined but errors in the diameter estimation will be rather large. In the case shown in 54A, the most robust diameter measurement may be performed.

Indeed, stable measurement of a blood vessel may be achieved when all relevant features are continuously accessible through the ultrasound data stream during a monitoring session. This means that motion of this blood vessel, either due to pulsatile flow nature or relative transducer motion with respect to the subject, should not affect rendering of these features by an algorithm. Additionally, the outcome of the measurement should not be strongly affected by the position, and reposition, of the patch at different location on the patient, by different operators.

Overall, it is clear that automatic selection, and if needed - real time adaptation - of the B-mode gain value would prove greatly beneficial for hemodynamic measurements and analysis.

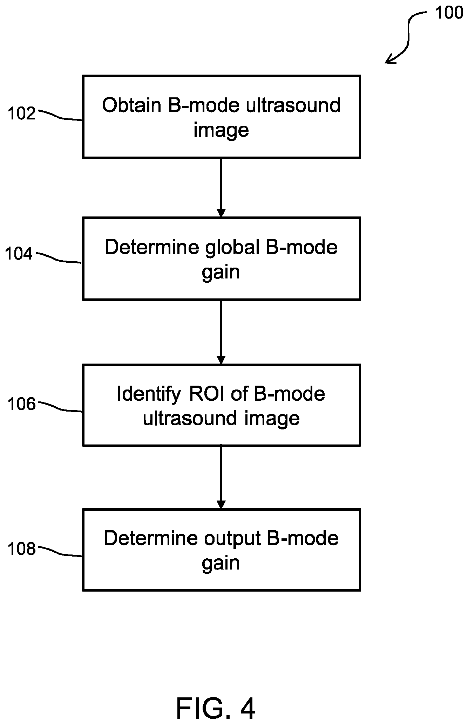

Moving onto Figure 4, there is presented a flow diagram 100 of a method for identifying a gain parameter value for ultrasound hemodynamic analysis according to an exemplary embodiment.

At step 102, a B-mode ultrasound image of a blood vessel is obtained. This may be obtained directly from an ultrasound monitor, may be acquired from a medical database, or otherwise. The B-mode ultrasound image may be an image including a cross-section of a blood vessel, or part of a blood vessel. The blood vessel may be the CCA of a subject.

At step 104, a global B-mode gain value which results in a global image entropy of the B-mode ultrasound image being within a target entropy range is determined. The target entropy range may be a range of entropies that relate to B-mode ultrasound images where a region of interest of the image may be identified. In other words, a B-mode gain value is identified which may be used to produce a B-mode ultrasound image containing enough information to identify a region of interest. The global B-mode gain may be determined using any known technique for global B-mode image gain optimization. Non-limiting example of such determination may be based, for instance, on a signal-to-noise ratio (SNR) of the B-mode ultrasound image or a comparison of the B-mode ultrasound image to a reference image.

In this way, a B-mode ultrasound image may be acquired which is suitable for further processing. Optimization of the global B-mode gain does not always lead to good conditions for hemodynamic analysis, as it may instead improve contrast of the surrounding tissue, rather than the blood vessel of interest. Therefore, additional steps are necessary in order to obtain a B-mode gain value that may deliver accurate and robust hemodynamic analysis. Specifically, further optimization which results in a clearer presentation of features of the blood vessel wall (such as an intima and media thickness) may greatly improve hemodynamic analysis.

At step 106, a region of interest of the B-mode ultrasound image resulting from the global B-mode gain value is identified. The region of interest may be an area of the image containing the blood vessel, including the vessel wall. If the B-mode ultrasound image contains multiple blood vessels, then the region of interest may relate to only one blood vessel, or may related to more than one blood vessel. The region of interest may be identified using a known method of image feature identification performed on the B-mode ultrasound image acquired using the global B-mode gain value.

At step 108, an output B-mode gain value based on a contrast-to-noise ratio of the region of interest is determined. Thus, an output B-mode gain value may be determined which might be used to produce a B-mode ultrasound image having a good quality in the region of interest, resulting in more accurate and robust hemodynamic analysis.

Additionally, the method may further comprise a step of checking if the global image entropy of the B-mode ultrasound image resulting from the output B-mode gain value is within the target entropy range. This may ensure that the output B-mode gain value has sufficient information for accurate hemodynamic analysis.

To paraphrase the above, initially the B-mode gain value may be adjusted for a suitable entropy of the full image, resulting in a global B-mode gain value. The global B-mode gain value must provide a B-mode ultrasound image with information enough to start the local optimization in the region of interest. It is known that, in general, overall B-mode image gain optimization does deliver the best settings for blood/wall regions of the blood vessels.

Then, a region of interest is identified for further improvement. In the region of interest, the image quality must be appropriate for blood vessel diameter sizing. Therefore, local image optimization is carried out on a blood vessel region of interest. This step specifically addresses the above problem. Overall, this optimization will ensure the capability of performing high quality feature extraction under dynamic conditions.

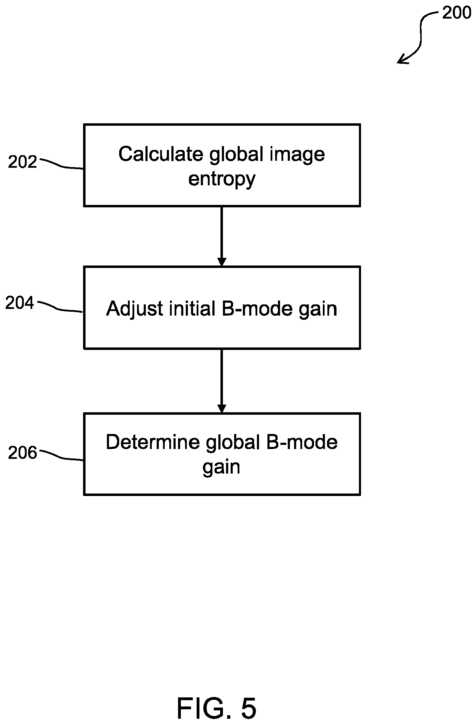

Figure 5 shows a flow diagram 200 of a method for determining a global B-mode gain according to an aspect of an exemplary embodiment. In this case, the obtained B-mode ultrasound image results from an initial B-mode gain value. The initial B-mode gain value may be a preset parameter value, as recommended by a vendor of tan ultrasound monitor, or may be a different initial value.

At step 202, the global image entropy of the B-mode ultrasound image may be calculated. This may be performed using known methods.

At step 204, the initial B-mode gain value may be adjusted. This may include increasing the global B-mode gain value from the initial B-mode gain value, or decreasing the global B-mode gain value from the initial B-mode gain value. The adjustment may be performed in steps, continuously, or randomly.

At step 206, the global B-mode gain value is determined based on the adjustment. The global B-mode gain results in the global image entropy of the B-mode ultrasound image being within the target entropy range. The target entropy range may be configured such that the global B-mode gain value produces a B-mode ultrasound with sufficient information such that a region of interest may be identified.

Practically, one method of realizing the above method may be the following:

A universal preset B-mode gain value for carotid ultrasound scan is applied to the ultrasound monitor; The entropy of the image with this universal preset is calculated.

If this entropy is outside the target entropy range, the B-mode gain value is either increased or decreased until the entropy of the ultrasound image is within the target entropy range.

After these steps, the image should have a good image entropy for further local optimization.

Accordingly, the global B-mode gain value may be determined which leads to global image optimization. This could also be performed in a number of different ways, for example by utilizing either direct or cumulative image histogram, comparing the current B-mode ultrasound image to a set of predefined high-quality B-mode images (a machine learning approach), or using other well-known in the literature techniques. Indeed, this step may be simplified as the image may be further improved in subsequent steps.

One simple method of determining the global B-mode gain value may comprise plotting a cumulative histogram of image entropy of the B-mode ultrasound image resulting from the initial B-mode gain value, and the determining the global B-mode gain value by adjusting the initial B-mode gain value, such that a log-average of the image entropy of the B-mode ultrasound image resulting from the global B-mode gain is within the target entropy range. Optionally, in this case, the target entropy range may be a range of log-averages of the cumulative histogram, the range of log-averages selected such that a common carotid artery, CCA, of corresponding B-mode ultrasound images may be detected.

This is just one of many realizations of methods for determining the global B-mode gain. Advantageously, this method may be used as a real time optimization algorithm.

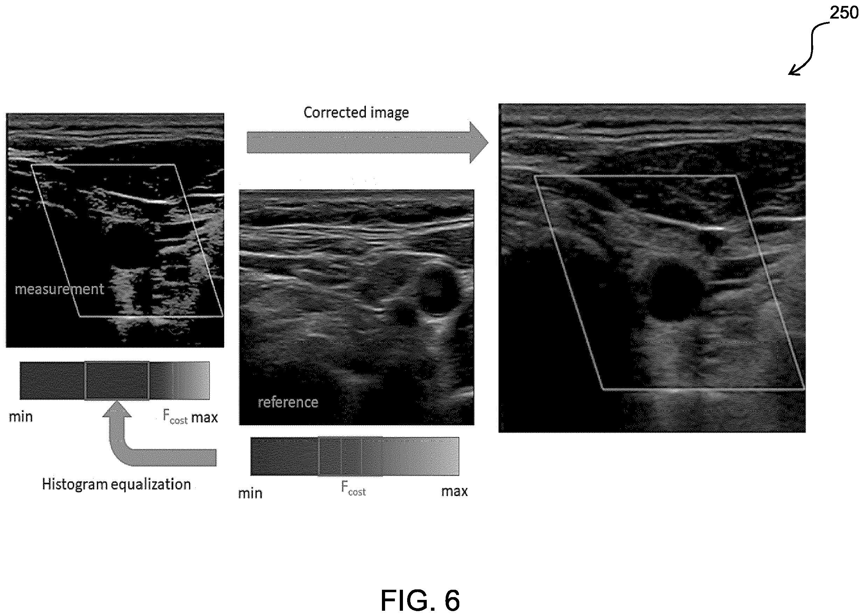

Figure 6 depicts various stages 250 of histogram equalization of an ultrasound image of a blood vessel responsive to changes in the global B-mode gain.

The use a log-cumulative histogram density of the log-compressed ultrasound data is one of the simplest estimators of global image entropy. The histogram may be normalized against the maximum intensity value. As the blood vessel region is of most interest, an offset may be used for the minimum gray values found in image to avoid singularities (for instance, we can use 1 as an offset leading to zero log-minimum reference point). The B-mode gain value may be gradually changed (increased/decreased) to have a log-average of the histogram in the target entropy range.

For example, a reference image, or a number of reference images, may exist with the cost function $F_{cost}$ representing global image entropy is within a target entropy range, the image may be considered to be a good starting point for the local optimization. Initially, the global entropy value may be outside of the target entropy range during the acquisition. If this is the case, a scan through different B-mode gain values is performed until $F_{cost}$ falls into the target entropy range.

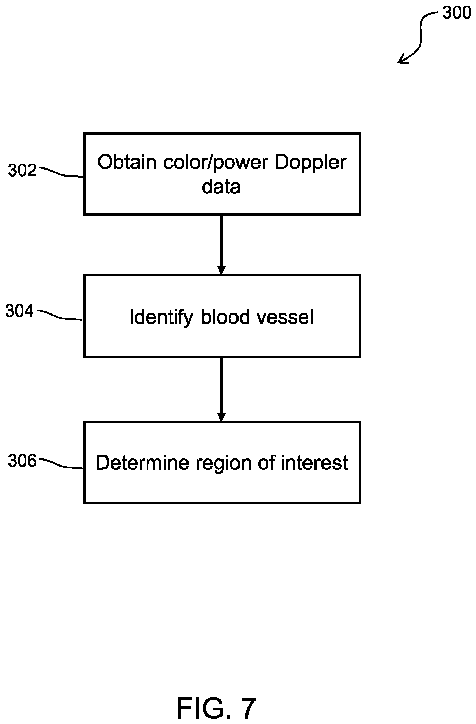

Figure 7 is a flow diagram 300 of a method for identifying a region of interest of an ultrasound image according to an aspect of an exemplary embodiment.

At step 302, color/power Doppler data corresponding to the B-mode ultrasound image resulting from the global B-mode gain value is obtained. Hemodynamic monitoring of the blood vessels usually involves color/power Doppler imaging, and therefore this obtaining data should require only a few, or no, additional steps.

At step 304, the blood vessel in the B-mode ultrasound image resulting from the global B-mode gain value is identified. This identification is based on the obtained color/power Doppler data. The identification may be performed using known methods.

At step 306, the region of interest is determined based on a diameter of the blood vessel and a direction of the blood vessel. The region of interest may correspond to an area of the image that is relevant for hemodynamic analysis, and so may include the blood vessel wall and interior.

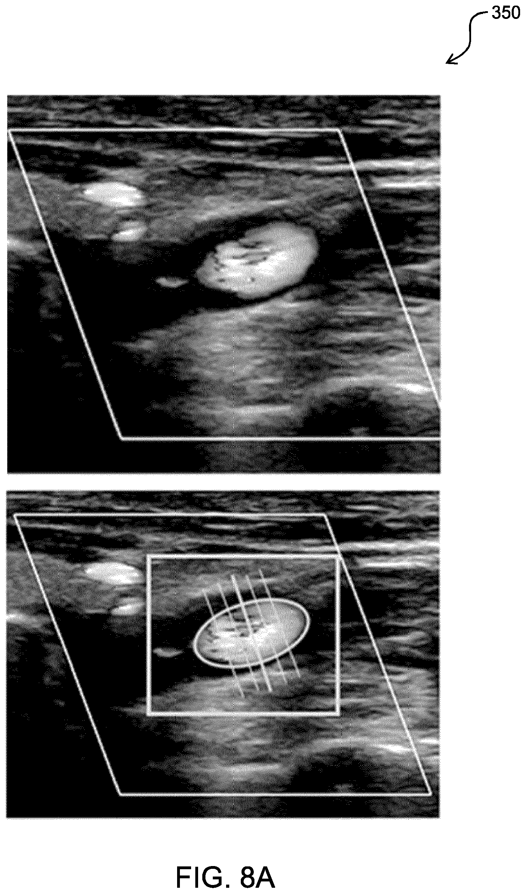

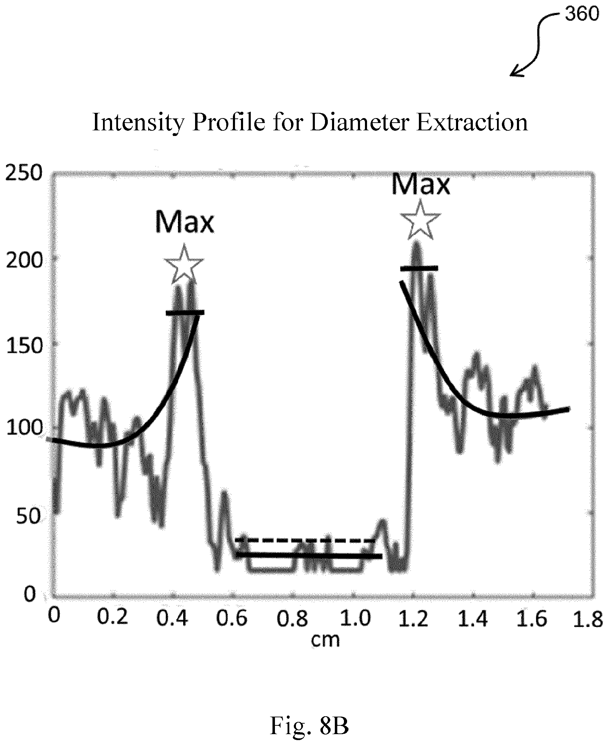

Turning to Figures 8A and 8B, the identification of a region of interest of an ultrasound image according to the method described in relation to Figure 7 is depicted.

First, a color/power Doppler algorithm is utilized for selecting the relevant blood vessel for further assessment of hemodynamic parameters. Once the blood vessel of interest is selected, a diameter measurement algorithm identifies the blood vessel direction and approximate blood vessel size. The final region of interest may be automatically specified by a bounding box, which is then used for subsequent local image optimization. It is the case that, for a reproducible diameter assessment of the blood vessel, a stable image quality along a central line of the blood vessel over multiple heart beats should be achieved.

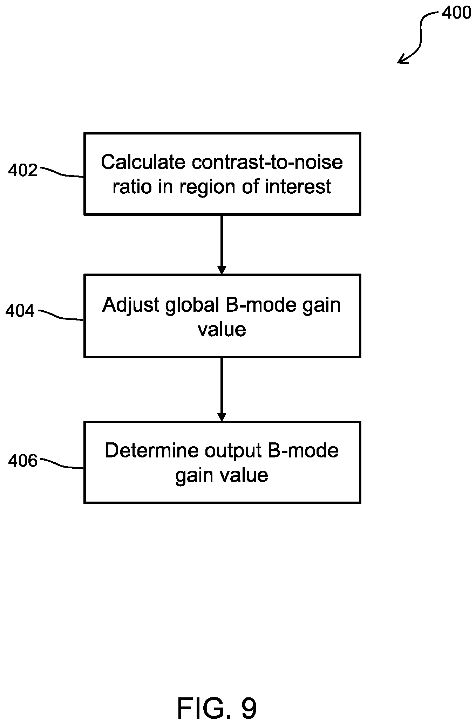

Figure 9 shows a flow diagram 400 of a method for determining an output B-mode gain value according to an aspect of an exemplary embodiment.

At step 402, the image parameter ratio of the region of interest of the B-mode ultrasound image resulting from the global B-mode gain value is calculated.

In an embodiment, the contrast-to-noise ratio of the region of interest of the B-mode ultrasound image resulting from the global B-mode gain value is calculated. The contrast-to-noise ratio is a good measure of the quality of the image at the region of interest, and is computationally inexpensive to calculate.

Calculating the contrast-to-noise ratio may be based on a difference between an average intensity of pixels in the region of interest corresponding to a tissue wall of the blood vessel and an average intensity of pixels in the region of interest corresponding to a background region. The calculating of the contrast-to-noise ratio may be further based on a variance of intensity of pixels in the background region of the region of interest.

By way of explanation, usually a contrast resolution according to Equation 1 is used as an indicator for quality of feature identification in ultrasound images.

$$CNR = \frac{|\mu_1 - \mu_2|}{\sqrt{\sigma_1{}^2 + \sigma_2{}^2}} \quad\quad (1)$$

where $\mu_1$ is the mean and variance of intensities of the feature pixels corresponding to tissue wall in the region of interest, respectively, and $\mu_2$ and $\sigma_2$ are the mean and variance of intensities of pixels in a background region (blood covered by ellipse region), respectively

A major main shortcoming of this definition is that it may be advantageous to have variance in the vessel wall (to capture the main features, such as intima/media transition), but minimize variance in the blood region.

Thus, the contrast to noise ratio may be calculated using Equation 2.

$$CNR = [\mu_1 - \mu_2] - \lambda \cdot \sigma_2 \quad\quad (2)$$

where $\lambda$ is a sensor specific parameter.

Moving onto step 404, the global B-mode gain value is adjusted. This may involve increasing or decreasing the gain value. The adjustment may be performed continuously, in steps, or randomly.

At step 406, the output B-mode gain value is determined, such that the output B-mode gain value results in a B-mode ultrasound image with an increased contrast-to-noise ratio in the region of interest. By increasing the contrast-to-noise ratio, the image quality in the region of interest may be improved, leading to more accurate and robust hemodynamic analysis of the blood vessel in the region of interest.

Optionally, determining the output B-mode gain value may include identifying a value of the output B-mode gain value that results in a maximized contrast to noise ratio in the region of interest. By maximizing the contrast-to-noise ratio, an image quality may be sufficient to provide accurate hemodynamic analysis. Indeed, maximizing the contrast-to-noise ratio may deliver stable feature measurement

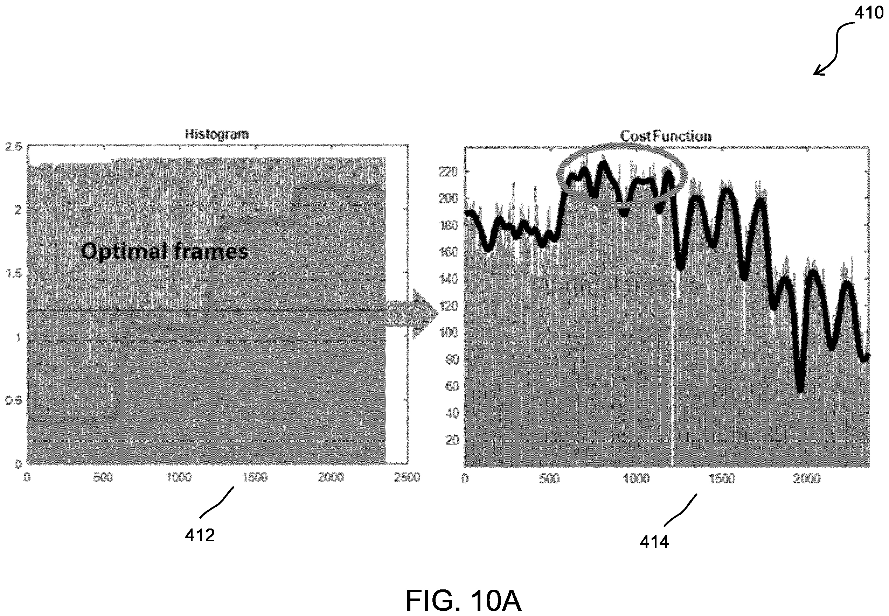

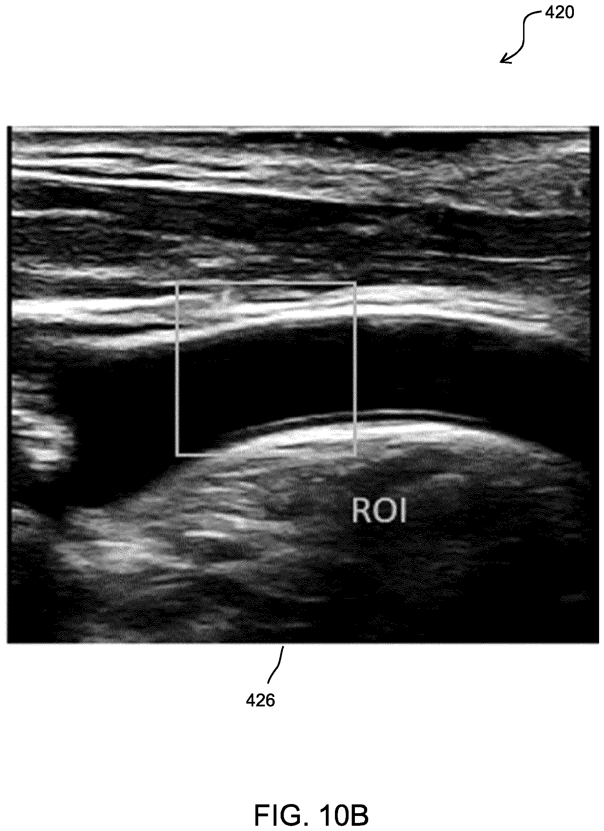

Figures 10A and 10B depict the impact of a change in the contrast-to noise ratio on the measurement of a diameter of a blood vessel. In other words, the behavior of the contrast-to-noise ratio as a function of the user gain is illustrated.

Initially, an optimal gain window is selected based on the cumulative image histogram 412. Then, the region of the maximum contrast to noise ratio within the optimal gain window 414 is identified, which leads to a suitable image 426 for hemodynamic analysis.

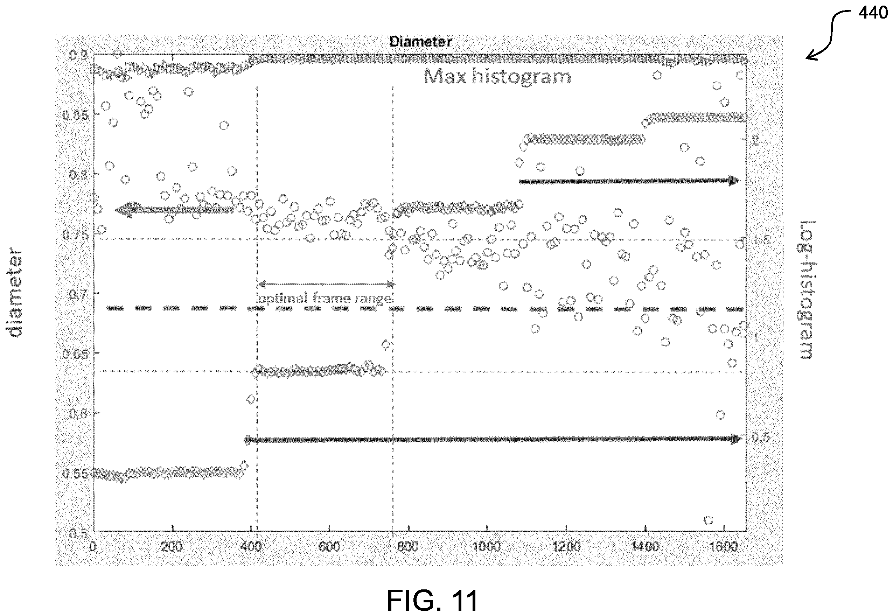

Figure 11 shows a graph 440 of the result of a measurement of a diameter of a blood vessel as a B-mode gain value is varied.

Overall, the graph demonstrates the importance in the proper identification of a B-mode gain value for hemodynamic analysis. Specifically, the graph shows that the most stable diameter measurement is within an optimal B-mode gain value window. Outside this B-mode gain window uncertainty and inaccuracy of the diameter measurement increases with change in B-mode gain. Indeed, during hemodynamic analysis, blood vessel diameter enters the expression for the blood flow quadratically. Thus, with an improperly adjusted B-mode gain, estimation of blood flow becomes much less accurate.

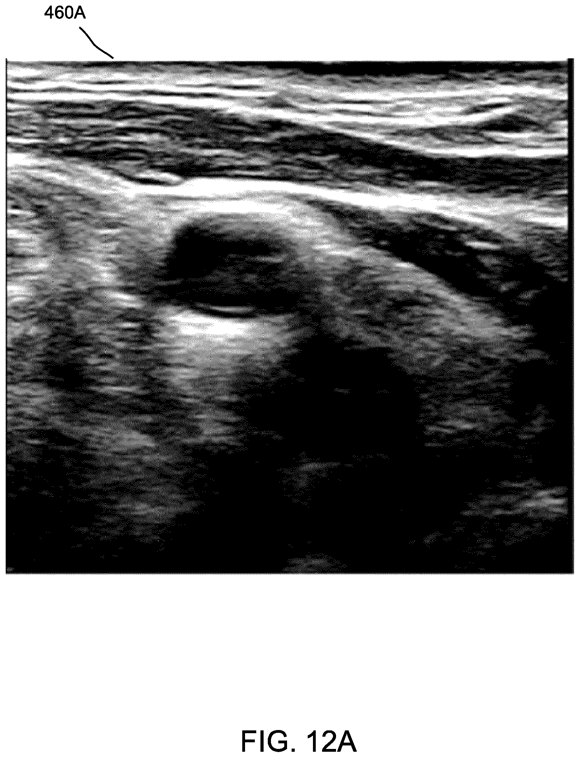

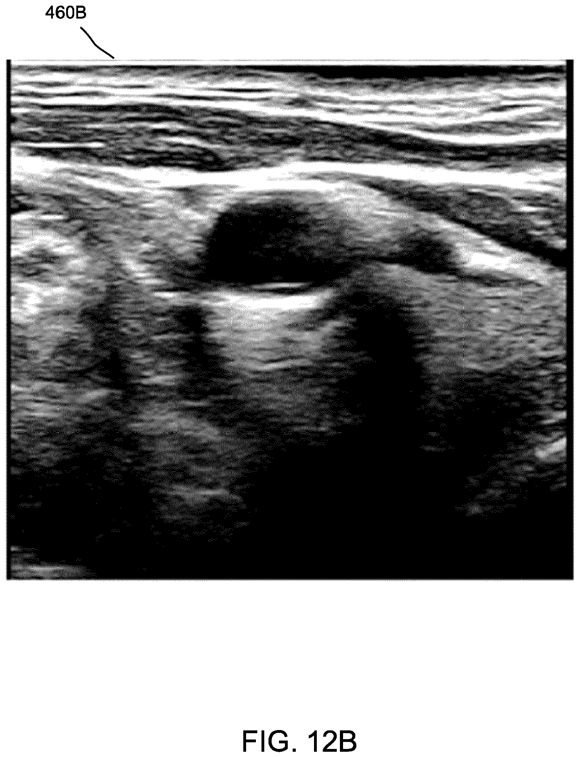

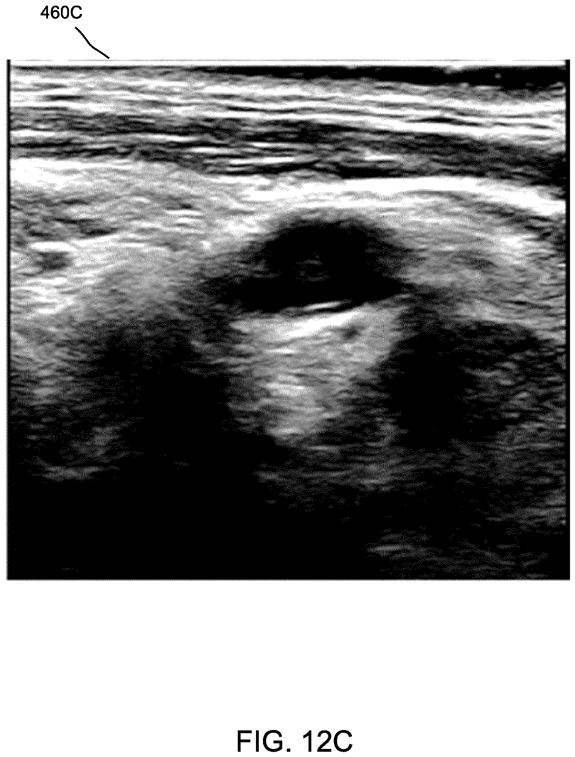

Figures 12A, 12B and 12C present B-mode ultrasound images 460A, 460B, and 460C acquired using two user defined B-mode gain values, and one using an automatically obtained output B-mode gain value.

In particular, 460A and 460B show images optimized by an operator for blood vessel diameter assessment. 460C shows an image acquired using an output B-mode gain acquired using a method according to an embodiment of the invention. As is clear from the images, the automatically optimized B-mode ultrasound image 460C is of the same/better quality than images 460A, 460B manually tuned by an experienced sonographer.

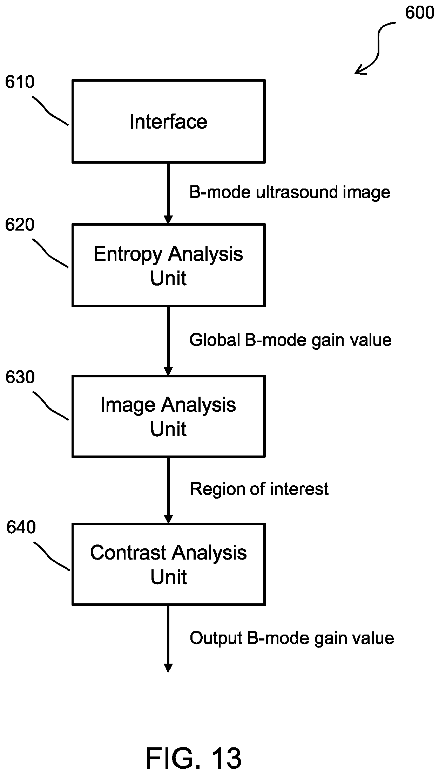

Turning to Figure 13, there is presented a simplified block diagram of a control system 600 for identifying a gain parameter value for ultrasound hemodynamic analysis according to an exemplary embodiment. The control system comprises an interface 610, an entropy analysis unit 620, an image analysis unit 630, and a contrast analysis unit 640.

The interface 610 is configured to obtain a B-mode ultrasound image of a blood vessel. The interface 610 may obtain the B-mode ultrasound image directly from a ultrasound unit, or may acquire the image from a medical database.

Further, the entropy analysis unit 620 is configured to determine a global B-mode gain value that results in a global image entropy of the B-mode ultrasound image being within a target entropy range. The entropy unit 620 may be configured to perform any known technique for assessing the entropy of an image, and for optimizing the B-mode gain for a whole image.

The image analysis unit 630 is configured to identify a region of interest of the B-mode ultrasound image resulting from the global B-mode gain value. The region of interest may be identified by the image analysis unit 630 by using color/power Doppler data corresponding to the B-mode ultrasound image.

Finally, the contrast analysis unit 640 is configured to determine an output B-mode gain value based on a contrast-to-noise ratio of the region of interest.

Overall, the control system 600 may be configured to perform any of the embodiments described above.

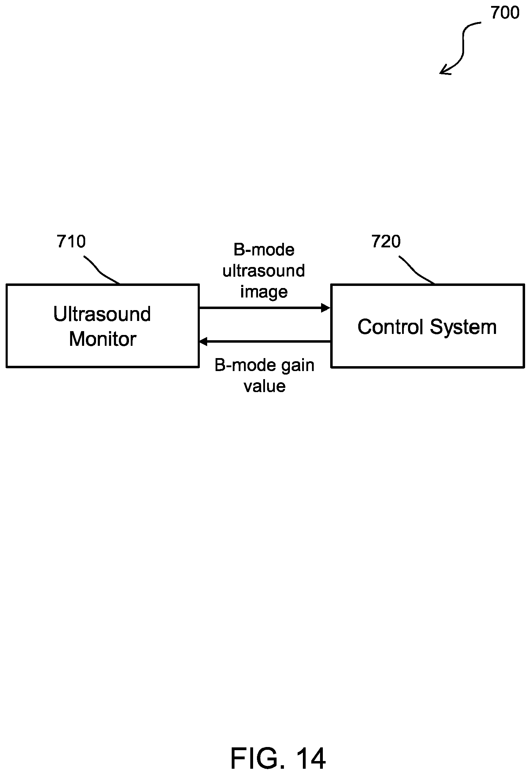

Figure 14 depicts a simplified block diagram of a system 700 for ultrasound hemodynamic analysis of a subject.

The system 700 may comprise the control system 600, 720 for identifying a gain parameter value as described in reference to Figure 13. The system 700 may further comprise an ultrasound monitor 710 configured to acquire a B-mode ultrasound image of a blood vessel of the subject at the output B-mode gain value determined by the control system.

The ultrasound monitor 710 may comprise a wearable ultrasound sensor or an ultrasound patch. Typical, wearable ultrasound monitors are less intrusive and uncomfortable compared to typical ultrasound monitors. As a result, subject comfort may be improved while undergoing hemodynamic analysis.

Further, the ultrasound monitor 710 may comprise a one dimensional ultrasonic array.

A single processor or other unit may fulfil the functions of several items recited in the claims.

A computer program product, which may be a collection of computer program code or instructions, may be stored/distributed on a suitable medium, such as an optical storage medium or a solid-state medium supplied together with or as part of other hardware, but may also be distributed in other forms, such as via the Internet, cloud-computing network or other wired or wireless telecommunication systems.

The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

The processing unit, for instance a controller implements the control method. The controller can be implemented in numerous ways; it may include a central processing unit (CPU), a digital signal processor (DSP), an ASIC, a controller, an FPGA, another hardware device, a firmware device, or any combination thereof configured to perform the operations described herein. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as random access memory (RAM), magneto-resistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. The storage media may be encoded with one or more programs that, when executed on one or more processors and/or controllers, perform at the required functions. Various storage media may be fixed within a processor or controller or may be transportable, such that the one or more programs stored thereon can be loaded into a processor or controller.

The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

While the invention has been illustrated and described in detail in the drawings and foregoing description, such illustration and description are to be considered illustrative or exemplary and not restrictive; the invention is not limited to the disclosed embodiments.

The mere fact that certain measures are recited in mutually different dependent claims does not indicate that a combination of these measures cannot be used to advantage.

Any reference signs in the claims should not be construed as limiting the scope.

**Claims**

1. A computer implemented method (100) for identifying a gain parameter value for ultrasound hemodynamic analysis, the method comprising:

   obtaining (102) a B-mode ultrasound image of a blood vessel; and
   determining (104) a global B-mode gain value which results in a global image entropy of the B-mode ultrasound image being within a target entropy range;
   **characterized by**
   identifying (106) a region of interest of the B-mode ultrasound image resulting from the global B-mode gain value; and
   determining (108) an output B-mode gain value based on a contrast-to-noise ratio of the region of interest.

2. The computer implemented method of claim 1, wherein the obtained B-mode ultrasound image results from an initial B-mode gain value, and wherein determining the global B-mode gain value comprises:

   calculating (202) the global image entropy of the B-mode ultrasound image; and
   determining (206) the global B-mode gain value by adjusting (204) the initial B-mode gain value, the global B-mode gain resulting in the global image entropy of the B-mode ultrasound image being within the target entropy range.

3. The computer implemented method of claim 2, wherein adjusting (204) the initial B-mode gain value to determine the global B-mode gain value comprises one of

   increasing the global B-mode gain value from the initial B-mode gain value until the global image entropy of the B-mode ultrasound image resulting from the B-mode gain value is within the target entropy range; or
   decreasing the global B-mode gain value from the initial B-mode gain value until the global image entropy of the B-mode ultrasound image resulting from the B-mode gain value is within the target range.

4. The computer implemented method of any preceding claim, wherein the obtained B-mode ultrasound image results from an initial B-mode gain value, and wherein determining the global B-mode gain value comprises:

   plotting a cumulative histogram of image entropy of the B-mode ultrasound image resulting from the initial B-mode gain value;
   determining the global B-mode gain value by adjusting the initial B-mode gain value, such that a log-average of the image entropy of the B-mode ultrasound image resulting from the global B-mode gain is within the target entropy range.

5. The computer implemented method of claim 4, wherein the target entropy range is a range of log-averages of the cumulative histogram, the range of log-averages selected such that a common carotid artery, CCA, of corresponding B-mode ultrasound images may be detected.

6. The computer implemented method of any preceding claim, wherein identifying the region of interest comprises:

obtaining (302) color/power Doppler data corresponding to the B-mode ultrasound image resulting from the global B-mode gain value;

identifying (304) the blood vessel in the B-mode ultrasound image resulting from the global B-mode gain value based on the obtained color/power Doppler data; and

determining (306) the region of interest based on a diameter of the blood vessel and a direction of the blood vessel.

7. The computer implemented method of any preceding claim, wherein determining the output B-mode gain value comprises:

calculating (402) the contrast-to-noise ratio of the region of interest of the B-mode ultrasound image resulting from the global B-mode gain value; and

determining (406) the output B-mode gain value by adjusting (404) the global B-mode gain value to result in an increase in the contrast to noise ratio of the region of interest.

8. The computer implemented method of claim 7, wherein determining the output B-mode gain value comprises: identifying a value of the output B-mode gain value which results in a maximized contrast to noise ratio in the region of interest.

9. The computer implemented method of claim 8, wherein calculating the contrast-to-noise ratio is based on a difference between an average intensity of pixels in the region of interest corresponding to a tissue wall of the blood vessel and an average intensity of pixels in the region of interest corresponding to a background region, and wherein calculating the contrast-to-noise ratio is further based on a variance of intensity of pixels in the background region of the region of interest.

10. The computer implemented method of any preceding claim, further comprising:
checking if the output B-mode gain value the global image entropy of the B-mode ultrasound image resulting from the output B-mode gain value is within the target entropy range.

11. A computer program product comprising computer readable code, the computer readable code being configured such that, on execution by a suitable computer or processor, the computer or processor is caused to perform the method as claimed in any of claims 1 to 10.

12. A control system (600) for identifying a gain parameter value for ultrasound hemodynamic analysis, the control system comprising:

an interface (610) configured to obtain a B-mode ultrasound image of a blood vessel; and

an entropy analysis unit (620) configured to determine a global B-mode gain value which results in a global image entropy of the B-mode ultrasound image being within a target entropy range;

**characterized by**

an image analysis unit (630) configured to identify a region of interest of the B-mode ultrasound image resulting from the global B-mode gain value; and

a contrast analysis unit (640) configured to determine an output B-mode gain value based on a contrast-to-noise ratio of the region of interest.

13. A system (700) for ultrasound hemodynamic analysis of a subject, the system comprising:

a control system (720) for identifying a gain parameter value, the control system being configured to carry out a method according to any of claims 1-10; and

an ultrasound monitor (710) configured to acquire a B-mode ultrasound image of a blood vessel of the subject at the output B-mode gain value determined by the control system.

14. The system of claim 13, wherein the ultrasound monitor (710) comprises a wearable ultrasound sensor or an ultrasound patch.

15. The system of claim 13 or 14, wherein the ultrasound monitor (710) comprises a one-dimensional ultrasonic array.

13

**Patentansprüche**

1. Computerimplementiertes Verfahren (100) zum Identifizieren eines Verstärkungsparameterwerts für hämodynamische Ultraschallanalyse, wobei das Verfahren umfasst:

   Erhalten (102) eines B-Mode-Ultraschallbildes eines Blutgefäßes; und
   Bestimmen (104) eines globalen B-Mode-Verstärkungswertes, der darin resultiert, dass eine globale Bildentropie des B-Mode-Ultraschallbildes innerhalb eines Zielentropiebereichs liegt;
   **gekennzeichnet durch**
   Identifizieren (106) eines Bereichs von Interesse des B-Mode-Ultraschallbildes, der aus dem globalen B-Mode-Verstärkungswert resultiert; und
   Bestimmen (108) eines Ausgabe-B-Mode-Verstärkungswertes auf der Grundlage eines Kontrast-Rausch-Verhältnisses des Bereichs von Interesse.

2. Computerimplementiertes Verfahren nach Anspruch 1, wobei das erhaltene B-Mode-Ultraschallbild aus einem anfänglichen B-Mode-Verstärkungswert resultiert, und wobei Bestimmen des globalen B-Mode-Verstärkungswerts umfasst:

   Berechnen (202) der globalen Bildentropie des B-Mode-Ultraschallbildes; und
   Bestimmen (206) des globalen B-Mode-Verstärkungswertes durch Anpassen (204) des anfänglichen B-Mode-Verstärkungswertes, wobei die globale B-Mode-Verstärkung darin resultiert, dass die globale Bildentropie des B-Mode-Ultraschallbildes innerhalb des Zielentropiebereichs liegt.

3. Computerimplementiertes Verfahren nach Anspruch 2, wobei Anpassen (204) des anfänglichen B-Mode-Verstärkungswertes zum Bestimmen des globalen B-Mode-Verstärkungswertes eines der Folgenden umfasst:

   Erhöhen des globalen B-Mode-Verstärkungswertes von dem anfänglichen B-Mode-Verstärkungswert, bis die globale Bildentropie des B-Mode-Ultraschallbildes, die aus dem B-Mode-Verstärkungswert resultiert, innerhalb des Zielentropiebereichs liegt; oder
   Verringern des globalen B-Mode-Verstärkungswertes von dem anfänglichen B-Mode-Verstärkungswert, bis die globale Bildentropie des B-Mode-Ultraschallbildes, die aus dem B-Mode-Verstärkungswert resultiert, innerhalb des Zielbereichs liegt.

4. Computerimplementiertes Verfahren nach einem vorstehenden Anspruch, wobei das erhaltene B-Mode-Ultraschallbild aus einem anfänglichen B-Mode-Verstärkungswert resultiert, und wobei Bestimmen des globalen B-Mode-Verstärkungswerts umfasst:

   Plotten eines kumulativen Histogramms der Bildentropie des B-Mode-Ultraschallbildes, das aus dem anfänglichen B-Mode-Verstärkungswert resultiert;
   Bestimmen des globalen B-Mode-Verstärkungswertes durch Anpassen des anfänglichen B-Mode-Verstärkungswertes, sodass ein logarithmischer Mittelwert der Bildentropie des B-Mode-Ultraschallbildes, der aus der globalen B-Mode-Verstärkung resultiert, innerhalb des Zielentropiebereichs liegt.

5. Computerimplementiertes Verfahren nach Anspruch 4, wobei der Zielentropiebereich ein Bereich von logarithmischen Mittelwerten des kumulativen Histogramms ist, wobei der Bereich der logarithmischen Mittelwerte so ausgewählt ist, dass eine gemeinsame Halsschlagader, CCA, von entsprechenden B-Mode-Ultraschallbildern erkannt werden kann.

6. Computerimplementiertes Verfahren nach einem vorstehenden Anspruch, wobei Identifizieren des Bereichs von Interesse umfasst:

   Erhalten (302) von Farb-/Power-Doppler-Daten, die dem B-Mode-Ultraschallbild entsprechen, das aus dem globalen B-Mode-Verstärkungswert resultiert;
   Identifizieren (304) des Blutgefäßes in dem B-Mode-Ultraschallbild, das aus dem globalen B-Mode-Verstärkungswert auf der Grundlage der erhaltenen Farb/Power-Doppler-Daten resultiert; und
   Bestimmen (306) des Bereichs von Interesse auf der Grundlage eines Durchmessers des Blutgefäßes und einer Richtung des Blutgefäßes.

7. Computerimplementiertes Verfahren nach einem vorstehenden Anspruch, wobei Bestimmen des Ausgabe-B-Mode-Verstärkungswerts umfasst:

Berechnen (402) des Kontrast-Rausch-Verhältnisses des Bereichs von Interesse des B-Mode-Ultraschallbildes, das aus dem globalen B-Mode-Verstärkungswert resultiert; und
Bestimmen (406) des Ausgabe-B-Mode-Verstärkungswertes durch Anpassen (404) des globalen B-Mode-Verstärkungswertes, um eine Erhöhung des Kontrast-Rausch-Verhältnisses des Bereichs von Interesse zu erreichen.

8. Computerimplementiertes Verfahren nach Anspruch 7, wobei Bestimmen des Ausgabe-B-Mode-Verstärkungswerts umfasst:
Identifizieren eines Ausgabe-B-Mode-Verstärkungswertes, der in einem maximalen Kontrast-Rausch-Verhältnis in dem Bereich von Interesse resultiert.

9. Computerimplementiertes Verfahren nach Anspruch 8, wobei Berechnen des Kontrast-Rausch-Verhältnisses auf der Grundlage einer Differenz zwischen einer durchschnittlichen Intensität von Pixeln in dem Bereich von Interesse, der einer Gewebewand des Blutgefäßes entspricht, und einer durchschnittlichen Intensität von Pixeln in dem Bereich von Interesse, der einem Hintergrundbereich entspricht, erfolgt, und wobei Berechnen des Kontrast-Rausch-Verhältnisses weiter auf der Grundlage einer Varianz der Intensität von Pixeln in dem Hintergrundbereich des Bereichs von Interesse erfolgt.

10. Computerimplementiertes Verfahren nach einem vorstehenden Anspruch, weiter umfassend:
Prüfen, ob der Ausgabe-B-Mode-Verstärkungswert die globale Bildentropie des B-Mode-Ultraschallbildes, die aus dem Ausgabe-B-Mode-Verstärkungswert resultiert, innerhalb des Zielentropiebereichs liegt.

11. Computerprogrammprodukt, das computerlesbaren Code umfasst, wobei der computerlesbare Code dazu konfiguriert ist, bei Ausführung durch einen geeigneten Computer oder Prozessor den Computer oder Prozessor zu veranlassen, das Verfahren nach einem der Ansprüche 1 bis 10 durchzuführen.

12. Steuersystem (600) zum Identifizieren eines Verstärkungsparameterwerts für hämodynamische Ultraschallanalyse, wobei das Steuersystem umfasst:

eine Schnittstelle (610), die dazu konfiguriert ist, ein B-Mode-Ultraschallbild eines Blutgefäßes zu erhalten; und
eine Entropie-Analyseeinheit (620), die dazu konfiguriert ist, einen globalen B-Mode-Verstärkungswert zu bestimmen, der darin resultiert, dass eine globale Bildentropie des B-Mode-Ultraschallbildes innerhalb eines Zielentropiebereichs liegt;
**gekennzeichnet durch**
eine Bildanalyseeinheit (630), die dazu konfiguriert ist, einen Bereich von Interesse des B-Mode-Ultraschallbildes zu identifizieren, der aus dem globalen B-Mode-Verstärkungswert resultiert; und
eine Kontrastanalyseeinheit (640), die dazu konfiguriert ist, einen Ausgabe-B-Mode-Verstärkungswert auf der Grundlage eines Kontrast-Rausch-Verhältnisses des Bereichs von Interesse zu bestimmen.

13. System (700) zur hämodynamischen Ultraschallanalyse einer Person, wobei das System umfasst:

ein Steuersystem (720) zum Identifizieren eines Verstärkungsparameterwerts, wobei das Steuersystem dazu konfiguriert ist, ein Verfahren nach einem der Ansprüche 1-10 durchzuführen; und
einen Ultraschallmonitor (710), der dazu konfiguriert ist, ein B-Mode-Ultraschallbild eines Blutgefäßes der Person mit dem von dem Steuersystem bestimmten Ausgabe-B-Mode-Verstärkungswert zu erhalten.

14. System nach Anspruch 13, wobei der Ultraschallmonitor (710) einen tragbaren Ultraschallsensor oder ein Ultraschall-Patch umfasst.

15. System nach Anspruch 13 oder 14, wobei der Ultraschallmonitor (710) ein eindimensionales Ultraschall-Array umfasst.

**Revendications**

1. Procédé mis en oeuvre par ordinateur (100) pour identifier une valeur de paramètre de gain pour une analyse hémodynamique par ultrasons, le procédé comprenant :

   l'obtention (102) d'une image ultrasonore en mode B d'un vaisseau sanguin ; et
   la détermination (104) d'une valeur de gain globale en mode B qui entraîne une entropie d'image globale de l'image ultrasonore en mode B se situant dans une plage d'entropie cible ;
   **caractérisé par**
   l'identification (106) d'une région d'intérêt de l'image ultrasonore en mode B résultant de la valeur de gain globale en mode B ; et
   la détermination (108) d'une valeur de gain de sortie en mode B sur la base d'un rapport contraste/bruit de la région d'intérêt.

2. Procédé mis en oeuvre par ordinateur selon la revendication 1, dans lequel l'image ultrasonore en mode B obtenue résulte d'une valeur de gain initiale en mode B, et dans lequel la détermination de la valeur de gain globale en mode B comprend :

   le calcul (202) de l'entropie d'image globale de l'image ultrasonore en mode B ; et
   la détermination (206) de la valeur de gain globale en mode B en ajustant (204) la valeur de gain initiale en mode B, le gain global en mode B entraînant l'entropie d'image globale de l'image ultrasonore en mode B se situant dans la plage d'entropie cible.

3. Procédé mis en oeuvre par ordinateur selon la revendication 2, dans lequel l'ajustement (204) de la valeur de gain initiale en mode B pour déterminer la valeur de gain globale en mode B comprend l'une des opérations suivantes :

   l'augmentation de la valeur de gain globale en mode B à partir de la valeur de gain initiale en mode B jusqu'à ce que l'entropie d'image globale de l'image ultrasonore en mode B résultant de la valeur de gain en mode B soit dans la plage d'entropie cible ; ou
   la diminution de la valeur de gain globale en mode B à partir de la valeur de gain initiale en mode B jusqu'à ce que l'entropie d'image globale de l'image ultrasonore en mode B résultant de la valeur de gain en mode B soit dans la plage cible.

4. Procédé mis en oeuvre par ordinateur selon une quelconque revendication précédente, dans lequel l'image ultrasonore en mode B obtenue résulte d'une valeur de gain initiale en mode B, et dans lequel la détermination de la valeur de gain globale en mode B comprend :

   le traçage d'un histogramme cumulatif de l'entropie d'image de l'image ultrasonore en mode B résultant de la valeur de gain initiale en mode B ;
   la détermination de la valeur de gain globale en mode B en ajustant la valeur de gain initiale en mode B, de telle sorte qu'une moyenne logarithmique de l'entropie d'image de l'image ultrasonore en mode B résultant du gain global en mode B soit dans la plage d'entropie cible.

5. Procédé mis en oeuvre par ordinateur selon la revendication 4, dans lequel la plage d'entropie cible est une plage de moyennes logarithmiques de l'histogramme cumulatif, la plage de moyennes logarithmiques étant sélectionnée de telle sorte qu'une artère carotide commune, CCA, d'images ultrasonores en mode B correspondantes puisse être détectée.

6. Procédé mis en oeuvre par ordinateur selon une quelconque revendication précédente, dans lequel l'identification de la région d'intérêt comprend :

   l'obtention (302) de données Doppler couleur/puissance correspondant à l'image ultrasonore en mode B résultant de la valeur de gain globale en mode B ;
   l'identification (304) du vaisseau sanguin dans l'image ultrasonore en mode B résultant de la valeur de gain globale en mode B sur la base des données Doppler couleur/puissance obtenues ; et
   la détermination (306) de la région d'intérêt sur la base d'un diamètre du vaisseau sanguin et d'une direction du vaisseau sanguin.

**7.** Procédé mis en oeuvre par ordinateur selon une quelconque revendication précédente, dans lequel la détermination de la valeur de gain de sortie en mode B comprend :

le calcul (402) du rapport contraste/bruit de la région d'intérêt de l'image ultrasonore en mode B résultant de la valeur de gain globale en mode B ; et
la détermination (406) de la valeur de gain de sortie en mode B en ajustant (404) la valeur de gain globale en mode B pour entraîner une augmentation du rapport contraste/bruit de la région d'intérêt.

**8.** Procédé mis en oeuvre par ordinateur selon la revendication 7, dans lequel la détermination de la valeur de gain de sortie en mode B comprend :
l'identification d'une valeur de la valeur de gain de sortie en mode B qui entraîne un rapport contraste/bruit maximisé dans la région d'intérêt.

**9.** Procédé mis en oeuvre par ordinateur selon la revendication 8, dans lequel le calcul du rapport contraste/bruit est basé sur une différence entre une intensité moyenne de pixels dans la région d'intérêt correspondant à une paroi tissulaire du vaisseau sanguin et une intensité moyenne de pixels dans la région d'intérêt correspondant à une région d'arrière-plan, et dans lequel le calcul du rapport contraste/bruit est en outre basé sur une variance d'intensité de pixels dans la région d'arrière-plan de la région d'intérêt.

**10.** Procédé mis en oeuvre par ordinateur selon une quelconque revendication précédente, comprenant en outre :
la vérification du fait que la valeur de gain de sortie en mode B l'entropie d'image globale de l'image ultrasonore en mode B résultant de la valeur de gain de sortie en mode B est ou non dans la plage d'entropie cible.

**11.** Produit de programme informatique comprenant un code lisible par ordinateur, le code lisible par ordinateur étant configuré de telle sorte que, lors de son exécution par un ordinateur ou un processeur approprié, l'ordinateur ou le processeur soit amené à effectuer le procédé selon l'une quelconque des revendications 1 à 10.

**12.** Système de commande (600) permettant d'identifier une valeur de paramètre de gain pour une analyse hémodynamique par ultrasons, le système de commande comprenant :

une interface (610) configurée pour obtenir une image ultrasonore en mode B d'un vaisseau sanguin ; et
une unité d'analyse d'entropie (620) configurée pour déterminer une valeur de gain globale en mode B qui entraîne une entropie d'image globale de l'image ultrasonore en mode B se situant dans une plage d'entropie cible ;
**caractérisé par**
une unité d'analyse d'image (630) configurée pour identifier une région d'intérêt de l'image ultrasonore en mode B résultant de la valeur de gain globale en mode B ; et
une unité d'analyse de contraste (640) configurée pour déterminer une valeur de gain de sortie en mode B sur la base d'un rapport contraste/bruit de la région d'intérêt.

**13.** Système (700) d'analyse hémodynamique par ultrasons d'un sujet, le système comprenant :

un système de commande (720) pour identifier une valeur de paramètre de gain, le système de commande étant configuré pour mettre en oeuvre un procédé selon l'une quelconque des revendications 1-10 ; et
un moniteur à ultrasons (710) configuré pour acquérir une image ultrasonore en mode B d'un vaisseau sanguin du sujet à la valeur de gain de sortie en mode B déterminée par le système de commande.

**14.** Système selon la revendication 13, dans lequel le moniteur à ultrasons (710) comprend un capteur à ultrasons portable ou un patch à ultrasons.

**15.** Système selon la revendication 13 ou 14, dans lequel le moniteur à ultrasons (710) comprend un réseau ultrasonore unidimensionnel.

FIG. 1

FIG. 2

FIG. 3A

Fig. 3B

52B

54B

56B

58B

50

100

```
        ┌─────────────────────────────┐
102 ────│   Obtain B-mode ultrasound  │
        │            image            │
        └─────────────────────────────┘
                      │
                      ▼
        ┌─────────────────────────────┐
104 ────│   Determine global B-mode   │
        │            gain             │
        └─────────────────────────────┘
                      │
                      ▼
        ┌─────────────────────────────┐
106 ────│     Identify ROI of B-mode  │
        │      ultrasound image       │
        └─────────────────────────────┘
                      │
                      ▼
        ┌─────────────────────────────┐
108 ────│   Determine output B-mode   │
        │            gain             │
        └─────────────────────────────┘
```

FIG. 4

200

202 —— Calculate global image entropy

204 —— Adjust initial B-mode gain

206 —— Determine global B-mode gain

FIG. 5

FIG. 6

300

```
302 ─⌒─  ┌─────────────────────────┐
            │ Obtain color/power Doppler │
            │          data            │
            └─────────────────────────┘
                        │
                        ▼
304 ─⌒─  ┌─────────────────────────┐
            │    Identify blood vessel   │
            └─────────────────────────┘
                        │
                        ▼
306 ─⌒─  ┌─────────────────────────┐
            │  Determine region of interest │
            └─────────────────────────┘
```

FIG. 7

350

FIG. 8A

Fig. 8B

400

402 — Calculate contrast-to-noise
ratio in region of interest

404 — Adjust global B-mode gain
value

406 — Determine output B-mode
gain value

FIG. 9

FIG. 10A

FIG. 10B

FIG. 11

460A

FIG. 12A

460B

FIG. 12B

460C

FIG. 12C

600

610

Interface

↓ B-mode ultrasound image

620

Entropy Analysis
Unit

↓ Global B-mode gain value

630

Image Analysis
Unit

↓ Region of interest

640

Contrast Analysis
Unit

↓ Output B-mode gain value

FIG. 13

700

| 710 | B-mode<br>ultrasound<br>image | 720 |
|---|---|---|
| Ultrasound<br>Monitor | | Control System |
| | B-mode gain<br>value | |

FIG. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080154131 A
- JP 2000139914 A
- US 20100069755 A
- US 20120123265 A
- US 20080242982 A

**Non-patent literature cited in the description**

- **MOSHAVEGH et al.** *Advanced Automated Gain Adjustments for In-Vivo Ultrasound Imaging,* 2015